# EUROPÄISCHE PATENTSCHRIFT

(11) **EP 2 745 801 B1**
(45) Veröffentlichungstag und Bekanntmachung des Hinweises auf die Patenterteilung: **21.02.2018**
(21) Anmeldenummer: 12197844.9
(22) Anmeldetag: 18.12.2012
(51) Int. Cl.: A61C 13/00, A61C 13/09, A61C 13/20

(54) **Verfahren zur Herstellung eines Dentalrestaurationsteils, sowie Dentalofen**
Method for producing a dental restoration part, and dental oven
Procédé destiné à la fabrication d'une pièce de restauration dentaire, ainsi que four dentaire

(43) Veröffentlichungstag der Anmeldung: 25.06.2014
(73) Patentinhaber: Ivoclar Vivadent AG, 9494 Schaan (LI)
(72) Erfinder: Lorünser, Johannes, 6700 Bludenz (AT); Brotzge, Michael, 6842 Koblach (AT); Grünenfelder, Robert, 9492 Eschen (LI); Kettner, Philipp, 6830 Rankweil (AT)
(74) Vertreter: Baronetzky, Klaus

(56) Entgegenhaltungen:
- EP-A1- 1 555 499
- EP-A1- 1 561 433
- EP-A1- 2 550 928
- EP-A1- 2 551 621
- WO-A1-00/08415

## Beschreibung

Die Erfindung betrifft ein Verfahren zur Herstellung eines Dentalrestaurationsteils, gemäß dem Oberbegriff von Anspruch 1, sowie einen Dentalofen, gemäß dem Oberbegriff von Anspruch 15.

Es ist seit längerem bekannt, dass die Qualität erzeugter Dentalrestaurationsteile davon abhängt, wie das herzustellende Dentalrestaurationsteil mit den Nachbarzähnen harmoniert. Dies gilt sowohl hinsichtlich der Form als auch der Farbe, aber auch beispielsweise des Farbverlaufs und der Transluszenz.

Um ein möglichst naturgetreues und harmonisches Erscheinungsbild des Dentalrestaurationsteils zu erzeugen, ist es bekannt geworden, mittels einer Kamera die Nachbarzähne aufzunehmen und das Erscheinungsbild des Dentalrestaurationsteils in Abhängigkeit hiervon zu erzeugen. Hierbei besteht der Nachteil, dass eine 2D-Kamera nur flächig arbeitet, so dass lediglich die Farbvarianz der Nachbarzähne bei dem Dentalrestaurationsteil abbildbar ist, nicht aber beispielsweise die Transluszenz.

Ferner ist es bekannt geworden, ein Dentalrestaurationsteil mittels CAD/CAM-Verfahren basierend auf den gewonnenen Daten zu erzeugen. Ein Beispiel hierfür lässt sich der DE 101 13 753 entnehmen. Auch ist es bekannt geworden, den zu erzeugenden Zahn oder das zu erzeugende Dentalrestaurationsteil virtuell in eine Abbildung der Nachbarzähne gleichsam einzufügen, um den Eindruck des zu erzeugenden Dentalrestaurationsteils besser abschätzen zu können. Ein derartiges Verfahren ist aus dem deutschen Patent 10 2004 002 724 B4 ersichtlich.

Aus der EP 1 561 433 A1 ist das Herstellen einer Dentalprothese ausgehend von einem physikalischen Modell, das mindestens eine Materialschicht aufweist, bekannt. Das Aufbringen der Materialschicht kann wiederholt werden, sofern ein Vergleich eines Ist-Bildes des Ist-Modells mit einem Soll-Bild ein Materialdefizit anzeigt.

EP 2 551 621 A1 offenbart einen Dentalofen für das Brennen von Dentalrestaurationen mit einer programmierbaren Steuervorrichtung für den Dentalofen, durch welche gesteuert der Ofen einen Brennzyklus durchführt, mit einer Eingabevorrichtung für die Steuervorrichtung, die einen berührungsempfindlichen Bildschirm aufweist, mit einer Bildaufnahmevorrichtung, die einen Bildaufnahmebereich aufweist, der außerhalb des Ofens angeordnet ist, wobei die Steuervorrichtung eine Speichervorrichtung zur Speicherung von Temperaturwerten aufweist. Sämtliche bekannten Verfahren sind bereits seit längerem vorgeschlagen oder veröffentlicht, aber das erreichte Ergebnis hängt stark von der Erfahrung und dem Können des mit der Herstellung des Dentalrestaurationsteils beauftragten Zahntechnikers ab.

Demgegenüber liegt der Erfindung die Aufgabe zugrunde, ein Verfahren zur Herstellung eines Dentalrestaurationsteils gemäß dem Oberbegriff von Anspruch 1 bzw. einen Dentalofen für die Herstellung eines derartigen Dentalrestaurationsteils, gemäß dem Oberbegriff von Anspruch 15 zu schaffen, die eine verbesserte Qualität der erzeugten Dentalrestaurationsteile ermöglichen.

Diese Aufgabe wird erfindungsgemäß durch Anspruch 1 gelöst. Vorteilhafte Weiterbildungen ergeben sich aus den Unteransprüchen.

Erfindungsgemäß wird die Herstellung eines Dentalrestaurationsteils gegliedert vorgenommen: Die äußere Kontur des herzustellenden Dentalrestaurationsteils wird durch dreidimensionale Erfassung der Nachbarzähne, beispielsweise über 3D-Kameras, festgelegt. Ferner wird - ebenfalls dreidimensional - der Unterbau festgelegt, und in einer Speichervorrichtung abgespeichert. Hierbei versteht es sich, dass die Speichervorrichtung vorab eine Vielzahl von möglichen Unterbaudaten abspeichern kann, die "passend" zu dem herzustellenden Dentalrestaurationsteil sind, während das dreidimensionale Oberflächenbild des Dentalrestaurationsteils von Fall zu Fall unterschiedlich ist.

Vorteilhafterweise lässt sich nun basierend auf den dreidimensionalen Oberflächendaten, die der herzustellenden Außenkontur des Dentalrestaurationsteils entsprechen, ein passender Unterbau auswählen.

Nachdem der Unterbau von der Außenkontur überall deutlich beabstandet ist, ist dessen optische Wirkung im Vergleich mit dem Dentalrestaurationsteil im Übrigen am geringsten.

Erfindungsgemäß wird nun der Differenzraum, also das dreidimensionale Gebilde zwischen dem Unterbau und der herzustellenden Außenkontur, in Schichten aufgeteilt, die aufzubringen sind.

Die Herstellung des Dentalrestaurationsteils wird nun so vorgenommen, dass während der Herstellung das Halbprodukt, also das Dentalrestaurationsteil vor seiner Fertigstellung, visuell erfasst wird, und zwar über eine Bildaufnahmevorrichtung. Die Bildaufnahmevorrichtung kann dann mindestens eine Schicht erfassen, und diese Schicht wird in vorteilhafter Ausgestaltung auch über eine Anzeigevorrichtung angezeigt. Die Erfassung erfolgt hierbei mindestens hinsichtlich der Schichtdicke der Schicht und der Farbe der Schicht über die Bildaufnahmevorrichtung praktisch automatisch.

Erfindungsgemäß wird nun die erfasste Schicht mindestens hinsichtlich der Schichtdicke mit den Soll-Bilddaten verglichen, und es wird ein Fehlersignal erzeugt, wenn die aufgebrachte Schicht hinsichtlich Schichtdicke und/oder Farbe und/oder anderer Parameter nicht der vorgegebenen Schicht gemäß den Bilddaten entspricht. Die Bilddaten bestehen insofern aus dreidimensionalen Daten zur herzustellenden Außenkontur des Dentalrestaurationsteils, aber auch aus dreidimensionalen Übergangsdaten, also Daten hinsichtlich der herzustellenden Schicht, basierend auf dem Unterbau bzw. der Differenz zwischen dem Unterbau und der Außenkontur.

Das Fehlersignal wird dann erzeugt, wenn die Abweichung zwischen dem vorgegebenen Schichtdaten gemäß den Bilddaten und der von der Bildaufnahmevorrichtung erfassten Werte einen Schwellwert übersteigt. Wenn die Abweichung geringer als der Schwellwert ist, wird kurzerhand die Schichtdicke oder ein sonstiger Parameter der nachfolgenden Schicht entsprechend angepasst.

Beispielhaft wird dies so vorgenommen, dass, wenn die Schichtdicke, die auf dem Unterbau aufgetragen ist, an einer Stelle 5 % größer als vorgegeben ist, die nachfolgende Schicht in ihrer Schichtdicke entsprechend angepasst wird, also um das den 5 % entsprechende Maß an dieser Stelle dünner hergestellt wird.

Überraschend lassen sich mit der erfindungsgemäßen Schichtdickenregelung oder Schichtparameterregelung der Aufbau eines Dentalrestaurationsteils wesentlich verbessern und insbesondere unabhängiger von dem Geschick und der Erfahrung des beauftragten Zahntechnikers machen. Der Zahntechniker bekommt automatisch bei der Erstellung des Dentalrestaurationsteils Hinweise, beispielsweise auf einer Anzeigevorrichtung, dass bestimmte Parameter an bestimmten Stellen nicht ganz korrekt sind, und es werden automatisch Hinweise eingeblendet, wie die entsprechenden Abweichungen bei der nächsten aufzubringenden Schicht zu korrigieren sind.

Dies gilt gleichermaßen für Farbfehler, die ebenfalls bei den unteren Schichten in gewissem Maße ausgeglichen bzw. kompensiert werden können, indem hinsichtlich der Farbe dann bei der nächstfolgenden Schicht gegengesteuert wird.

In vorteilhafter Ausgestaltung ist eine Nachführeinrichtung vorgesehen, die die Bildaufnahmevorrichtung bei der Aufnahme des Dentalrestaurationsteils während seiner Entstehung nachführt, so dass stets die dreidimensional erfasste Oberfläche der betreffenden Schicht im Fokus der Bildaufnahmevorrichtung liegt.

Die Anzeige auf der Anzeigevorrichtung zur Darstellung der aufgebrachten Schicht kann in beliebiger Art und Weise erfolgen, bevorzugt sowohl in Form von Linien oder Hüllkurven als auch über numerische Werte.

In weiterer vorteilhafter Ausgestaltung ist eine Datenbrille oder ein head mounted display als Anzeigevorrichtung vorgesehen, die dann gleich die Korrekturen für die nächsten aufzubringenden Schichten oder die nächste aufzubringende Schicht im Sinne einer gemischten Realität in das von der dreidimensionalen Kamera aufgenommene Bild einblendet.

Das erfindungsgemäße Verfahren lässt sich sowohl bei keramischen oder Komposit-Dentalrestaurationsteilen, aber auch bei zu polymerisierenden Dentalrestaurationsteilen einsetzen, und es ist günstig, dass das erfindungsgemäße Verfahren iterativ realisierbar ist, so dass mit der gleichen Ausrüstung von Schicht zu Schicht die gleichen Schritte bis zur letzten Schicht realisierbar sind.

Sowohl im Dentalofen als auch beim Polymerisieren erfolgt typischerweise eine Schrumpfung des aufgebrachten Dentalrestaurationsmaterials. Dies lässt sich bevorzugt je kompensieren, und zwar entweder, indem die aktuelle Schicht in einem gewissen Übermaß aufgetragen wird, oder indem für die nächste Schicht ein Übermaß einkalkuliert wird, um die Schrumpfung zu kompensieren.

Es versteht sich, dass das Aufbringen der Schichten gerade beim Einsatz von zu polymerisierenden Materialien auch je abwechselnd mit Polymerisationsschritten erfolgen kann, so dass das Schrumpfungsergebnis durch die erfindungsgemäße Bildaufnahmevorrichtung kompensierbar ist.

Bei Realisierung des Dentalrestaurationsteils als in einem Dentalofen zu brennendes Dentalrestaurationsteil lässt sich auch eine Programmsteuerung des Ofens basierend auf dem Vergleichsergebnis des Vergleichs zwischen dem aufgenommenen Bild des herzustellenden Dentalrestaurationsteils und den Bilddaten vornehmen, die vorab in dem Dentalofen oder getrennt von diesem abgespeichert sind, insbesondere in einer Speichervorrichtung, die eine Datenbank aufweist, und es lässt sich auch eine Sicherheitsfunktion einbauen, die ein Einschalten des Ofens verhindert, wenn die Divergenz zwischen dem aufgenommenen Bild der Bildaufnahmevorrichtung und dem abgespeicherten Bild ein vorgegebenes Maß übersteigt.

Hierbei lässt sich die Abweichung entweder automatisch durch Bildauswertung erfassen, oder hilfsweise auch durch visuelle Beurteilung.

Es ist auch möglich, die beiden Bilder elektronisch übereinander zu legen und über eine Differenzeinfärbung die Unterschiede deutlich zu machen, um dem Benutzer Hinweise hinsichtlich der Abweichungen zu geben und eine entsprechende Beurteilung hinsichtlich der weiteren, zu erbringenden Schritte zu erlauben.

Weitere Vorteile, Einzelheiten und Merkmale ergeben sich aus der nachfolgenden Beschreibung eines Ausführungsbeispiels der Erfindung anhand der Zeichnung.

Es zeigen:
- Fig. 1: eine schematische Ansicht eines erfindungsgemäßen Dentalofens, zur beispielhaften Ausführung eines erfindungsgemäßen Verfahrens;
- Fig. 2: eine schematische Darstellung einer erfindungsgemäßen Bildaufnahmevorrichtung mit dem partiell hergestellten Dentalrestaurationsteil; und
- Fig. 3: eine schematisch dargestellte Funktion des erfindungsgemäßen Verfahrens.

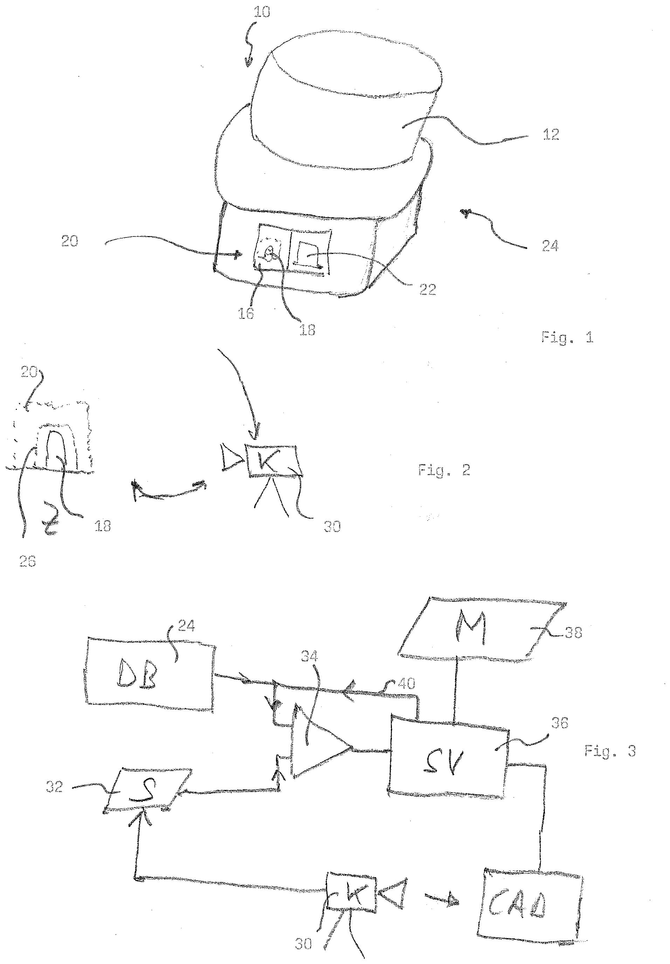

Der in Fig. 1 dargestellte Dentalofen 10 weist in der dargestellten Ausführungsform eine Ofenhaube 12 und ein Ofenunterteil 14 auf, an dem die Ofenhaube 12 in an sich bekannter Weise schwenkbar gelagert ist.

Das Ofenunterteil 14 trägt an seiner Vorderseite in der Darstellung gemäß Fig. 1 eine Anzeigevorrichtung 16. Die Anzeigevorrichtung 16 wird erfindungsgemäß eingesetzt, wobei in dem dargestellten Betriebszustand im linken Bereich ein Unterbau 18 einer Dentalrestauration durchgezogen dargestellt ist, und ein darauf aufzubringender Zahn als Dentalrestauration 20 in gestrichelter Form.

Demgegenüber ist in dem rechten Bereich der Anzeigevorrichtung 16 ein Zahn 22 dargestellt, dessen Bild aus einer Bildaufnahmevorrichtung gewonnen ist, wobei die Nachbarzähne des herzustellenden Zahns 20 dargestellt sind.

Diese Anzeige weist dem Zahntechniker dem Grunde nach den Weg, in welchen erfindungsgemäßen Schichten er den Unterbau 18 aufbauen muss, um zu dem Zahn 22 zu gelangen.

Während hier das erfindungsgemäße Verfahren anhand eines Dentalofens dargestellt ist, versteht es sich, dass in gleicher Weise die Erfindung auch die Herstellung eines Dentalrestaurationsteils mittels eines Polymerisationsgeräts erlaubt.

In Fig. 2 ist in schematischer Weise der Zahn Z 20 dargestellt, in diesem Fall mit seiner Außenkontur gestrichelt. Der Unterbau 18 ist wiederum mit einer durchgezogenen Linie dargestellt. In elektronischer Form liegen beide Darstellungen in dreidimensionaler Form vor, also als Hüllkurve im Raum, wobei die Daten des Unterbaus in einer Speichervorrichtung 24 nach der Art einer Datenbank abgespeichert sind, um den Aufbau der Schichten im einzelnen zu erleichtern. Erfindungsgemäß wird nun eine erste Schicht, deren Umrisse 26 in Fig. 1 schematisch dargestellt sind, auf den Unterbau 18 aufgebracht.

Dies kann in beliebiger geeigneter Weise erfolgen, beispielsweise auch über eines der bekannten Rapid-Prototyping-Verfahren, wobei die Schichtdicke sich dann aus der Anzahl und Anordnung der aufgebrachten Voxel ergibt.

Die Schichtdicke der Schicht mit dem Umriss 26 wird in geeigneter Weise vorab festgelegt, beispielsweise durch Berechnung der räumlichen Differenz zwischen dem Unterbau 18 und der Außenkontur 20 des Zahns. Es versteht sich, dass es bevorzugt ist, dass jede Schicht im Wesentlichen die gleiche Schichtstärke aufweist, allein schon, um bei der Polymerisation eine gleichmäßige Durchhärtung rasch realisieren zu können. Wenn beispielsweise die Aufbringung von drei Schichten entsprechend Fig. 2 erforderlich ist, um die erwünschte Schichtstärke und damit die Außenkontur 20 zu erreichen, wird die Schichtstärke der ersten Schicht 26 auf etwa ein Drittel der Gesamtschichtstärke festgelegt.

Die tatsächlich erzielte Schichtstärke und deren dreidimensionale Außenkontur wird erfindungsgemäß durch eine Bildaufnahmevorrichtung 30 ermittelt, die in Form einer dreidimensionalen Kamera ausgebildet ist, oder aber auch einer Kamera, die in alle Raumachsen verschwenkbar ist und aus der ein dreidimensionales Bild gewinnbar ist.

Aus Fig. 3 ist ersichtlich, dass zunächst basierend auf einer Speichervorrichtung 24 Grunddaten bereitgestellt werden, etwa des Unterbaus 18 oder aber beispielsweise Daten der Umgebung, in die der Zahn aufgebracht werden soll. Zugleich wird ein Bild 32 der Kamera 30 angezeigt, das diese aus den Nachbarzähnen gewonnen hat und das beispielsweise die Soll-Außenkontur des zu erzeugenden Dentalrestaurationsteils widerspiegelt, oder aber auch ein Bild des Zahns 22.

Die entsprechenden Bilddaten werden elektronisch ausgewertet und aufbereitet, so dass ein dreidimensionaler Vergleich möglich ist. Hierzu ist gemäß Fig. 3 eine Vergleichsvorrichtung 34 vorgesehen, die selbstverständlich auch in elektronischer Form per Software realisierbar ist und die gleichsam einen Soll-/Ist-Vergleich macht.

Im ersten Schritt ergibt sich aus dem Vergleich ein großer Unterscheid zwischen Soll- und Ist.

Mit der Steuervorrichtung 36 wird jedoch nicht der Bearbeitungsschritt vorgegeben, diesen Unterschied gleichsam in einem Zuge auszufüllen. Vielmehr erzeugt die Steuervorrichtung auf einem Monitor 38 ein Soll-Bild für die erste zu erzeugende Schicht, die von der Steuervorrichtung basierend auf einem Soll-/Ist-Vergleich und einer entsprechenden Unterteilung in einen ganzzahligen Wert, beispielsweise 3, oder aber auch 8, erzeugt wird.

Dementsprechend unterscheidet sich bei einer gewählten Schichtanzahl von 8 das auf dem Monitor 38 dargestellte Soll-Bild nur um weniges von dem Ist-Bild des Unterbaus 18 in dem ersten Verfahrensschritt, auf welchen die Kamera für den Abgleich zwischen Soll- und Ist gerichtet wird.

Die Anzahl der Schichten lässt sich vom Zahntechniker frei wählen, wobei hinsichtlich des gewählten Materials und der demit zu erreichenden Schichtstärken Vorgaben von der Steuervorrichtung 36 gemacht werden. Wenn das zu polymerisierende Material beispielsweise für eine maximale Schichtstärke von 1 mm spezifiziert ist, und ein Molar muss mit einer Gesamtschichtstärke von 4mm realisiert werden, werden für die Eingabe durch den Benutzer alle Werte von 1 bis 3 gesperrt, so dass lediglich eine Schichtenanzahl von 4 bis z. B. 10 einstellbar ist.

Die Einstellung und Interaktion mit dem Benutzer kann hierbei über die Anzeigevorrichtung 16 realisiert sein, die dann als Touchscreen ausgebildet ist, oder aber in an sich bekannter Weise über separate Bedientasten, beispielsweise rechts und links der Anzeigevorrichtung 16 in Fig. 1, oder einer entsprechenden Anzeige im Falle der Verwendung eines Polymerisationsgeräts.

Nachdem die Schichtdicke, aber auch die Materialwahl, der Farbton und die Farbvarianz, aber auch weitere chromatische Parameter, einschließlich der Transparenz, vorgegeben sind, erfolgt die Aufbringung der erwünschten Schicht bevorzugt über ein CAD/CAM-System, wie es aus Fig. 3 ersichtlich ist, das von der Steuervorrichtung 36 gesteuert wird.

Es versteht sich, dass es anstelle dessen auch möglich ist, die Schicht nach der Vorgabe der Steuervorrichtung manuell aufzubringen.

Während des Aufbringens, aber spätestens beim Beenden der Aufbringung der betreffenden Schicht, ist die Bildaufnahmevorrichtung 30 wie vorstehend dargelegt als dreidimensionale Bildaufnahmevorrichtung auf das Halbprodukt des Dentalrestaurationsteils gerichtet, so dass permanent, oder zumindest in regelmäßig wiederkehrenden Zeitpunkten, der Fortschritt der Aufbringung kontrolliert wird.

In diesem nächsten Schritt wird dann in einem Soll-Ist-Vergleich ein Vergleich mit der aus der Steuervorrichtung 36 abgezweigten Vorgabe gemacht, wozu die Verbindungslinie 40 in Fig. 3 dargestellt ist.

Die Legung der nächsten Schicht, deren Schichtstärke, deren Farbe und ähnlicher Parameter erfolgt dann im nächsten Schritt wiederum in der Steuervorrichtung 36. Dieser liegt der Soll-/Ist-Vergleich der Vergleichsvorrichtung 34 vor, und die Steuervorrichtung 36 erzeugt hieraus eine Abweichung vom Soll des ursprünglich geplanten Schichtaufbaus. Wenn sich beispielsweise herausstellt, dass die vorige Schicht an einer Stelle - beispielsweise nach dem Polymerisieren - um 5 % zu dünn ist, wird die nachfolgende Schicht an dieser Stelle um 5 % stärker gemacht, so dass insofern eine Kompensation erfolgt.

Abgesehen von der Kombination der Schichtstärke bzw. der dort induzierten Fehler erfolgt aber auch eine Kompensation weiterer Parameter, sowei dies technisch möglich ist. Wenn beispielsweise eine Schicht mit einem zu transparenten oder zu wenig dunklem Material realisiert ist, gegenüber dem ursprünglich geplanten Schichtaufbau, kann die nächste Schicht mit einem opakeren bzw. dunklerem Material realisiert werden, ohne dass das ästhetische Gesamterscheinungsbild wesentlich verändert ist. Dies gilt in besonderem Maße für die unteren Schichten.

In gleicher Weise wie hier bei der ersten und zweiten Schicht beschrieben, erfolgt die geregelte Schichterzeugung auch für die weiteren Schichten. Erfindungsgemäß sin mindestens zwei Schichten vorgesehen, es ist jedoch auch möglich, eine Vielzahl von Schichten wie beispielsweise zehn Schichten einzusetzen.

Während hier Kamera und Monitor, aber auch die Anzeigevorrichtung 16 gemäß Fig. 1, als separate Baueinheiten dargestellt sind, versteht es sich, dass entweder der Monitor und die Kamera oder die Anzeigevorrichtung 16 und die Kamera 30 in geeigneter Weise mit sogenannten Multimediabrillen zusammengefasst sein können, die nach der Art einer virtuellen Realität das anzuzeigende Bild in das natürliche Bild der Dentalrestauration einblenden.

Es versteht sich, dass als Schichten die an sich bekannten Schichten realisiert werden können, oder aber auch Sub-Schichten der an sich bekannten Schichten. Zu den an sich bekannten Schichten gehört die Opakerschicht, die Dentinschicht und die Enamelschicht (Zahnschmelzschicht), die je einzeln oder insgesamt in Subschichten unterteilbar sind.

## Patentansprüche

1. Verfahren zur Herstellung eines Dentalrestaurationsteils (20) mittels eines Dentalofens, welcher eine programmierbare Steuervorrichtung, eine Bildaufnahmevorrichtung (30), eine Speichervorrichtung (24) sowie eine Anzeigevorrichtung (16) aufweist, wobei das Dentalrestaurationsteil auf einem Unterbau (18) unter Aufbringung von mindestens zwei Schichten hergestellt wird, wobei Ausgangsdaten, insbesondere dreidimensionale Daten, des Unterbaus, in der Speichervorrichtung (24) abgespeichert werden, und Bilddaten (32), insbesondere dreidimensionale Daten, zur herzustellenden Außenkontur des Dentalrestaurationsteils und der aufzubringenden Schichten des Dentalrestaurationsteils, festgelegt und insbesondere abgespeichert werden, wobei das Dentalrestaurationsteil wenigstens teilweise während seiner Herstellung im Bildaufnahmebereich der Bildaufnahmevorrichtung (30) ist, wobei vor der Fertigstellung des Dentalrestaurationsteils (20) und nach Aufbringen mindestens einer Schicht, diese mindestens hinsichtlich ihrer Schichtdicke und/oder Farbe erfasst wird und über die Anzeigevorrichtung (16) angezeigt wird, wobei die erfasste Schicht mindestens hinsichtlich der Schichtdicke mit den Bilddaten (32) verglichen wird und ein Fehlersignal erzeugt wird, wenn die aufgebrachte Schicht hinsichtlich ihrer Schichtdicke und/oder Farbe und/oder der weiteren chromatischen Parameter, einschließlich der Transluzenz, nicht der vorgegebenen Schicht gemäß den Bilddaten (32) entspricht.

2. Verfahren nach Anspruch 1, **dadurch gekennzeichnet, dass** der Vergleich durch eine Vergleichsvorrichtung zwischen in der Speichervorrichtung (24) abgespeicherten Schichtdicken und den erfassten Schichtdicken erfolgt, insbesondere an einer Vielzahl von überwachten Stellen der dreidimensionalen Oberfläche der aufgebrachten Schicht.

3. Verfahren nach einem der vorhergehenden Ansprüche, **dadurch gekennzeichnet, dass** die Bestimmung der tatsächlich aufgebrachten Schichtdicke an jeder Stelle durch Differenzberechnung erfolgt, wobei eine Differenz zwischen der Kontur des erfassten Bildes nach Aufbringen der Schicht und der Kontur des Bildes vor Aufbringen der Schicht für die Berechnung zugrundegelegt wird, und zwar an jeder der überwachten Stellen.

4. Verfahren nach einem der vorhergehenden Ansprüche, **dadurch gekennzeichnet, dass** die Anzeigevorrichtung (16) das Fehlersignal abgibt, wenn der Unterschied zwischen der erfassten Schichtdicke und der gemäß dem Schichtungsprogramm in der Speichervorrichtung (24) abgelegten Schichtdicke größer als ein vorgegebener Schwellwert ist.

5. Verfahren gemäß einem der vorhergehenden Ansprüche, **dadurch gekennzeichnet, dass** die Anzeigevorrichtung (16) insbesondere als Datenbrille oder head mounted display ausgebildet ist und die gemäß dem Schichtungsprogramm erwünschte Schichtdicke oder weitere Einzelheiten der nächsten aufzubringenden Schicht aus weiterem Dentalmaterial in das von der Kamera (30) aufgenommene Bild im Sinne einer gemischten Realität einblendet.

6. Verfahren nach einem der vorhergehenden Ansprüche, **dadurch gekennzeichnet, dass** die von der Bildaufnahmevorrichtung (30) erfasste Schicht in mindestens zwei sich dreidimensional erstreckende Abschnitte unterteilt wird, wobei jeder der Abschnitte von der Vergleichsvorrichtung (34) mit in der Speichervorrichtung (24) abgespeicherten Daten verglichen wird.

7. Verfahren nach einem der vorhergehenden Ansprüche, **dadurch gekennzeichnet, dass** nach dem Aufbringen einer Schicht diese zusammen mit dem Unterbau (18) in einem Brennofen gebrannt wird oder mittels eines Polymerisationsgeräts polymerisiert wird und dass die weiteren Schichten iterativ nach dem gleichen Verfahren aufgebracht werden.

8. Verfahren nach einem der vorhergehenden Ansprüche, **dadurch gekennzeichnet, dass** nach dem Erfassen des Dentalrestaurationsteils (20) mit mindestens einer aufgebrachten Schicht mittels der Bildaufnahmevorrichtung, und insbesondere nach dem Brennen oder Polymerisieren dieses halbfertigen Dentalrestaurationsteils (20), über die Vergleichsvorrichtung ein Vergleich mit in der Speichervorrichtung abgespeicherten Schichtdicken des zu erstellenden Dentalrestaurationsteils (20) erfolgt.

9. Verfahren nach Anspruch 8, **dadurch gekennzeichnet, dass** eine Anzeigevorrichtung die Differenz zwischen dem erfassten Schichtdickenverlauf entsprechend der Kontur des halbfertigen Dentalrestaurationsteils (20) und der vorgegebenen Außenkontur des fertigen Dentalrestaurationsteils (20) gemäß den Daten der Speichervorrichtung (24) anzeigt und insbesondere die letzte Schicht, die zur Bereitstellung des Dentalrestaurationsteils (20) erforderlich ist, mit einem Übermaß gefertigt wird, das das Schrumpfen beim Brennen oder Polymerisieren im Wesentlichen vollständig kompensiert.

10. Verfahren nach einem der vorhergehenden Ansprüche, **dadurch gekennzeichnet, dass** bei Aufbringung der nächsten Schicht eine Abweichung zwischen den vorgegebenen, in der Speichervorrichtung (24) abgespeicherten Schichtdaten und erfassten Schichtdaten der zuvor aufgebrachten Schicht berücksichtigt wird, indem die Schichtdicke und/oder die Farbe der nächsten Schicht angepasst wird.

11. Verfahren nach einem der vorhergehenden Ansprüche, **dadurch gekennzeichnet, dass** eine beim Schichtaufbau fertiggestellte Schicht durch die Bildaufnahmevorrichtung (30) auch hinsichtlich ihrer Farbe erfasst wird und mit der in der Speichervorrichtung (24) abgespeicherten erwünschten Farbe verglichen wird und dass bei einer Farbdifferenz, die einen vorgegebenen Schwellenwert überschreitet, die Erstellung des Dentalrestaurationsteils (20) abgebrochen wird.

12. Verfahren nach einem der vorhergehenden Ansprüche, **dadurch gekennzeichnet, dass** die Anzeigevorrichtung (12) zusätzlich zu dem Dentalrestaurationsteil (20) im aktuellen Zustand, also nach Aufbringung der letzten Schicht, die Kontur der ersten (26) und der weiteren Schichten, gemäß den in der Speichervorrichtung (24) abgespeicherten Daten anzeigt.

13. Verfahren nach einem der vorhergehenden Ansprüche, **dadurch gekennzeichnet, dass** die Bildaufnahmevorrichtung (30) eine Nachführeinrichtung aufweist, die die Relativposition zwischen ihr und dem Dentalrestaurationsteil (20) während der Herstellung des Dentalrestaurationsteils (20) bei jeder Bildaufnahme der Bildaufnahmevorrichtung (30) gleich hält, insbesondere hinsichtlich des Abstandes, des Drehwinkels und/oder der Beleuchtung.

14. Verfahren nach einem der vorhergehenden Ansprüche, **dadurch gekennzeichnet, dass** die Anzeigevorrichtung (16) den Verlauf der aufgebrachten Schicht in Form von Linien oder Hüllkurven wiedergibt, und insbesondere auch über numerische Werte.

15. Dentalofen für das Brennen und/oder Pressen von Dentalrestaurationen (20), mit einer programmierbaren Steuervorrichtung für den Dentalofen, durch welche gesteuert der Ofen einen Brennzyklus oder einen Presszyklus durchführt, mit einer Eingabevorrichtung für die Steuervorrichtung, die gegebenenfalls einen berührungsempfindlichen Bildschirm aufweist, mit einer Bildaufnahmevorrichtung, die einen Bildaufnahmebereich aufweist, der außerhalb des Ofens angeordnet ist, wobei die Steuervorrichtung eine Speichervorrichtung (24), insbesondere eine Datenbank, aufweist, in welcher Bilder von Produkten für die Dentalrestaurationen in elektronischer Form abgespeichert sind, und eine Vergleichsvorrichtung aufweist, die eingerichtet ist, ein vor der Fertigstellung der Dentalrestauration (20) und nach Aufbringen mindestens einer Schicht eines Dentalrestaurationsteils über die Bildaufnahmevorrichtung (30) aufgenommenes Bild mit den abgespeicherten Bildern (32) zu vergleichen, wobei bei fehlender Übereinstimmung ein Fehlersignal erzeugbar ist und bei hinreichender Übereinstimmung, der Ofen gemäß einem zu dem Produkt passenden Programm steuerbar ist.

16. Dentalofen nach Anspruch 15, **dadurch gekennzeichnet, dass** die Vergleichsvorrichtung bei der hinreichenden Übereinstimmung auf einer Anzeigevorrichtung (16) des Dentalofens dem Benutzer das passende Programm anbietet, und dass das Programm bei Bestätigung durch den Benutzer startet.

## Claims

1. A method for the manufacture of a dental restoration part (20) by way of a dental furnace, comprising a programmable control device, an image acquisition device (30), a storage device (24) as well as a display device (16), wherein the dental restoration part Is manufactured on a sub-structure (18) by applying at least two layers, wherein initial data, especially three-dimensional data, of the sub-structure are stored in the storage device (24), and image data (32), especially three-dimensional data, for the outer contour to be manufactured of the dental restoration part and the layers to be manufactured of the dental restoration part, will be defined and especially will be stored, wherein the dental restoration part, during manufacture thereof, is at least partially located in the image acquisition zone of the acquisition device (30), wherein prior to completion of the dental restoration part (20) and following application of at least one layer, that layer is identified at least regarding its layer thickness and/or color and is displayed via the display device (16), wherein the layer identified is compared, at least regarding the layer thickness, to the image data (32) and an error signal is generated if the layer applied, regarding its layer thickness and/or color and/or the other chromatic parameters, Including translucence, does not correspond to the layer prescribed according to the image data (32).

2. The method according to Claim 1, **characterized in that** comparison is performed, by a comparison device, between the layer thicknesses stored in the storage device (24) and the layer thicknesses acquired, especially at a plurality of locations monitored of the three-dimensional surface of the applied layer.

3. The method according to one of the preceding Claims, **characterized in that** determination of the layer thickness actually applied on each location is done by difference calculating, wherein a difference between the contour of the acquired image following application of the layer and the contour of the image prior to application of the layer underlies the calculation, and underlies the calculation at each location monitored.

4. The method according to one of the preceding Claims, **characterized in that** the display device (16) releases the error signal, if the difference between the layer thickness acquired and the layer thickness deposited in the storage device (24) according to the layering program is larger than a prescribed threshold value.

5. The method according to one of the preceding Claims, **characterized In that** the display device (16), is formed especially as a data template or is formed as a head mounted display, and blends in the layer thickness desired according to the layering program or further details of the next layer to be applied of another dental material to the image acquired by the camera (30) in the sense of a mixed reality.

6. The method according to one of the preceding Claims, **characterized in that** the layer acquired by the image acquisition device (30) Is divided Into at least two three-dimensionally extending portions, wherein each portion from the comparison device (34) is compared to data stored in the storage device (24).

7. The method according to one of the preceding Claims, **characterized in that** following application of a layer, that layer, together with the sub-structure (18), is baked in a firing furnace or is polymerized by way of a polymerization device and **in that** the other layers are iteratively applied according to the same method.

8. The method according to one of the preceding Claims, **characterized in that** following identification of the dental restoration part (20) having at least one layer applied by means of the image acquisition device, and especially following baking or polymerization of this semi-finished dental restoration part (20), comparison, via the comparison device, to the layer thicknesses stored in the storage device of the dental restoration part (20) to be created is done.

9. The method according to Claim 8, **characterized in that** a display device displays the difference between the layer thickness profile corresponding to the contour of the semi-finished dental restoration part (20) and the prescribed outer contour of the final dental restoration part (20), according to the data of the storage device (24), and especially the last layer, which is required for providing the dental restoration part (20), is fabricated in an oversized way, essentially completely compensating shrinkage during firing or polymerizing.

10. The method according to one of the preceding Claims, **characterized in that** upon application of the next layer deviation between the prescribed layer data stored in the storage device (24) and layer data acquired of the previously applied layer is taken into account by adapting the layer thickness and/or the color of the next layer.

11. The method according to one of the preceding Claims, **characterized in that** a layer completed during layer building is acquired by the Image acquisition device (30), even in regard to its color, and is compared to the desired color stored in der storage device (24), and **in that** with a color difference exceeding a prescribed threshold value, preparation of the dental restoration part (20) is discontinued.

12. The method according to one of the preceding Claims, **characterized in that** the display device (12), in addition to the actual state dental restoration part (20, i.e, following application of the last layer, displays the contour of the first (26) and further layers according to the data stored in der storage device (24).

13. The method according to one of the preceding Claims, **characterized in that** the image acquisition device (30) comprises a tracking device, keeping equal the relative position between said tracking device and the dental restoration part (20) during manufacture of the dental restoration part (20) in each image acquisition of the image acquisition device (30), especially in regard of the distance, rotary angle and/or illumination.

14. The method according to one of the preceding Claims, **characterized in that** the display device (16) reflects the course of the applied layer in the form of lines or envelope curves and especially also by numerical values.

15. A dental furnace for firing and/or pressing dental restorations (20), comprising a programmable control device for the dental furnace, by which control of the control device the furnace performs a firing cycle or a pressing cycle, with an input device for the control device eventually comprising a touch sensitive screen, an image acquisition device, comprising an image acquisition zone which is arranged outside the furnace, wherein the control device comprises a storage device (24), especially a data base, In which images of products for the dental restorations are electronically stored, and comprises a comparison device, which Is configured to compare an Image acquired prior to completion of the dental restoration (20) and following application of at least one layer of a dental restoration part via the image acquisition device (30) to the image stored (32), wherein upon lack of correspondence an error signal is able to be generated and upon sufficient matching the furnace is controllable according to a program matching the product.

16. The dental furnace according to Claim 15, **characterized in that** the comparison device, upon sufficient correspondence, offers the appropriate program to the user on a display device (16) of the dental furnace, starting the program upon user actuation.

## Revendications

1. Procédé de fabrication d'une pièce de restauration dentaire (20) à l'aide d'un four dentaire, qui présente un dispositif de commande programmable, un dispositif de capture d'image (30), un dispositif de stockage (24), ainsi qu'un dispositif d'affichage (16), où la pièce de restauration dentaire est fabriquée sur un support (18) avec l'application d'au moins deux couches, où des données de sortie, en particulier des données tridimensionnelles du support sont sauvegardées dans le dispositif de stockage (24) et des données d'image (32), en particulier des données tridimensionnelles concernant le contour extérieur à fabriquer de la pièce de restauration dentaire et des couches à appliquer de la pièce de restauration dentaire sont définies et en particulier sauvegardées, où au cours de sa fabrication la pièce de restauration dentaire est au moins partiellement dans la zone de capture d'image du dispositif de prise d'image (30), où avant l'achèvement de la pièce de restauration dentaire (20) et après l'application d'au moins une couche, celle-ci est détectée au moins en ce qui concerne son épaisseur et/ou sa couleur est affichée sur le dispositif d'affichage (16), où la couche détectée est comparée au moins en ce qui concerné son épaisseur avec les données d'image (32) et un signal d'erreur est généré si la couche appliquée ne correspond pas à la couche prédéfinie selon les données d'image (32) en ce qui concerne son épaisseur et/ou sa couleur et/ou ses autres paramètres chromatiques, y compris la translucidité.

2. Procédé selon la revendication 1, **caractérisée en ce que** la comparaison est effectuée par un dispositif de comparaison entre l'épaisseur de couche détectée dans le dispositif de stockage (24) et l'épaisseur de couche détectée en particulier à une multitude de positions surveillées de la surface tridimensionnelle de la couche appliquée.

3. Procédé selon l'une des revendications précédentes, **caractérisé en ce que** l'épaisseur de couche effectivement appliquée à chaque position est déterminée par un calcul différentiel, où une différence entre le contour de l'image capturée après l'application de la couche et du contour de l'image avant l'application de la couche sert de base pour le calcul à chacune des positions surveillées.

4. Procédé selon l'une des revendications précédentes, **caractérisé en ce que** le dispositif d'affichage (16) donne le signal d'erreur, si la différence entre l'épaisseur de couche détectée et l'épaisseur de couche stockée dans le dispositif de stockage (24) selon le programme de stratification est supérieure à une valeur de seuil prédéterminé.

5. Procédé selon une des revendications précédentes, **caractérisé en ce que** le dispositif d'affichage (16) est formé en particulier comme lunettes à réalité augmentée ou visiocasque et superpose l'épaisseur de couche souhaitée selon le programme de stratification ou d'autres détails de la couche suivante à être appliquée à partir de matériel dentaire supplémentaire dans l'image enregistrée par la caméra (30) comme réalité mixte.

6. Procédé selon l'une des revendications précédentes, **caractérisé en ce que** la couche détectée par le dispositif de capture d'image (30) est divisée en au moins deux sections s'étendant en trois dimensions, où chacune des sections est comparée par le dispositif de comparaison (34) avec des données stockées dans le dispositif de stockage (24).

7. Procédé selon l'une des revendications précédentes, **caractérisé en ce qu'**après l'application d'une couche celle-ci est culte dans un four avec le support (18) ou est polymérisé au moyen d'un appareil de polymérisation et que les autres couches sont appliquées de manière itérative selon la même procédure.

8. Procédé selon l'une des revendications précédentes, **caractérisé en ce que**, après la détection de la pièce de restauration dentaire (20) avec au moins une couche appliquée au moyen du dispositif de capture d'images et surtout après la cuisson ou polymérisation de cette pièce de restauration dentaire (20) semi-finie, une comparaison avec les épaisseurs de couche de la pièce de restauration dentaire (20) en cours de fabrication enregistrées dans le dispositif de stockage a lieu à travers le dispositif de comparaison.

9. Procédé selon la revendication 8, **caractérisé en ce qu'**un dispositif d'affichage affiche la différence entre le tracé d'épaisseurs de couche correspondant au contour de la pièce de restauration dentaire (20) semi-finie et le contour extérieur prédéfinie de la pièce de restauration dentaire (20) finie selon les données du dispositif de stockage (24) et en particulier la dernière couche, qui est nécessaire pour la fourniture de la pièce de restauration dentaire (20), est fabriquée avec un surdimensionnement, qui compense essentiellement totalement le rétrécissement qui survient lors de la cuisson ou la polymérisation.

10. Procédé selon l'une des revendications précédentes, **caractérisé en ce que** lors de l'application de la couche suivante un écart entre les données prédéfinies enregistrées dans le dispositif de stockage (24), et les données de couche détectées des couches appliquées précédemment est pris en compte, en ajustant l'épaisseur et/ou la couleur de la couche suivante.

11. Procédé selon l'une des revendications précédentes, **caractérisé en ce que** lors de la construction de la structure en couches une couche achevée est aussi capturé par le dispositif de capture d'image (30) en ce qui concerne sa couleur et est comparé avec la couleur souhaitée enregistrée dans le dispositif de stockage (24) et qu'en cas d'une différence de couleur qui dépasse un seuil donné, la création de la pièce de restauration dentaire (20) est interrompue.

12. Procédé selon l'une des revendications précédentes, **caractérisé en ce que** le dispositif d'affichage (12) en plus de la pièce de restauration dentaire (20) dans son état actuel, c'est-à-dire après l'application de la dernière couche, affiche le contour de la première (26) et les autres couches, selon les données enregistrées dans le dispositif de stockage (24).

13. Procédé selon l'une des revendications précédentes, **caractérisé en ce que** le dispositif de capture d'image (30) présente un dispositif de suivi, qui maintient la position relative entre celle-ci et la pièce de restauration dentaire (20) au cours de la fabrication de la pièce de restauration dentaire (20) pour chaque image capturée par le dispositif de capture d'image (30), en particulier en ce qui concerne la distance, l'angle de rotation et/ou l'éclairage.

14. Procédé selon l'une des revendications précédentes, **caractérisé en ce que** le dispositif d'affichage (16) reflète le tracé de la couche appliquée sous forme de lignes ou d'enveloppes et en particulier aussi par des valeurs numériques.

15. Four dentaire pour cuisson et/ou en pressage de restaurations dentaires (20 four), avec un dispositif de commande programmable pour le four dentaire, commandé par lequel le four effectue un cycle de cuisson ou un cycle de pressage, avec un dispositif d'entrée pour le dispositif de commande, qui présente éventuellement un écran tactile, avec un dispositif de capture d'image, qui a une zone de capture d'image, qui est agencé à l'extérieur du four, où le dispositif de commande présente un dispositif de stockage (24), en particulier une base de données qui, dans laquelle des images de produits pour les restaurations dentaires sont stockées sous forme électronique, et présente un dispositif de comparaison, qui est aménagé pour comparer une image capturée par le dispositif de capture d'image (30) avant l'achèvement de la restauration dentaire (20) et après l'application d'au moins une couche d'une pièce de restauration dentaire avec les images enregistrées (32), où en cas de défaut de conformité, un signal d'erreur peut être produit et en cas de conformité suffisante, le four peut être commandé conformément à un programme approprié pour le produit.

16. Four dentaire selon la revendication 15, **caractérisé en ce que** le dispositif de comparaison en cas de conformité suffisante propose à l'utilisateur sur un dispositif d'affichage (16) du four dentaire le programme approprié et que le programme démarre en cas de confirmation par l'utilisateur.
